# EUROPEAN PATENT SPECIFICATION

(11) **EP 3 719 111 B1**
(45) Date of publication and mention of the grant of the patent: **20.10.2021**
(21) Application number: 19382250.9
(22) Date of filing: 04.04.2019
(51) Int. Cl.: C12M 1/12, B01L 3/00, C12M 1/22, C12M 1/00

(54) **DEVICES FOR CELLULARIZED MEMBRANE CULTIVATION AND KITS**
VORRICHTUNGEN FÜR ZELLULARISIERTE MEMBRANKULTIVIERUNG UND KITS
DISPOSITIFS DE CULTURE DE MEMBRANE CELLULARISÉE ET KITS

(43) Date of publication of application: 07.10.2020
(73) Proprietor: Universidad de Navarra, 31009 Pamplona (Navarra) (ES); Leartiker, S.Coop., 48270 Markina-Xemein (ES)
(72) Inventor: ANDREÚ OLTRA, Enrique José, 31008 PAMPLONA (ES); LARREATEGI MAKATZAGA, Pablo, 48270 MARKINA-XEMEIN (ES); PRÓSPER CARDOSO, Felipe, 31008 PAMPLONA (ES); ZALDUA HUICI, Ane Miren, 48270 MARKINA-XEMEIN (ES)
(74) Representative: ZBM Patents - Zea, Barlocci & Markvardsen

(56) References cited:
- EP-A1- 0 171 854
- JP-A- 2001 299 326
- US-A- 5 462 874
- US-A1- 2014 162 352

## Description

The present disclosure relates to devices for cultivating cells and tissue growth. The present disclosure further relates to kits including such devices.

### BACKGROUND

For laboratory requirements, it is known to raise or cultivate cell cultures in a shallow culture vessel, the cells simply being placed in the culture vessel and culture medium being added. A cover may then be removably mounted to the culture vessel for selectively enclosing an interior of the cell culture vessel. Such devices for cell cultivation may also be provided with a biocompatible membrane (either natural or synthetic), placed inside the device, for cell cultivation and tissue growth.

Cellularized membranes may be used e.g. in tissue engineering. Tissue engineering particularly refers to the use of cell cultivation to improve or replace biological tissues e.g. to repair or replace portions of bone, cartilage, blood vessel, skin, muscle or other biological tissue.

Cell cultivation in this case may be carried out in a controlled and sterile environment in a cleanroom. After tissue has been grown, it needs to be transported to the operating room where it may be implanted in a patient.

It has been found that with known devices, the membranes tend to shrink after nutritional fluid has been added to it. In this respect, a membrane for cell cultivation and tissue growth which is not properly stretched i.e. a shrunk membrane may not allow the cells to suitably adhere to the membrane and grow.

Additionally, the cell cultures inside the vessels may be exposed to pollution by environmental agents during handling and e.g. transport to the operating room, because containers are often open. Or they can be designed to be closed using the above-commented cover, which however does not necessarily provide complete sealing. In this respect, the cover may accidentally be removed. Sterility is thus not guaranteed once the tissue leaves the cleanroom.

It is also known to use a closed device e.g. a sterile flask comprising a receptacle with a lid or closure piece. However, a problem with this kind of flasks is that accessing the interior can be time-consuming and it may be a cumbersome task to carry out. Particularly, removing the cells from the interior of such devices may be very laborious and time-consuming. A further problem may be that such closed devices cannot easily exchange gases, particularly oxygen, with the cells, especially when these are placed in areas of the closed systems that are far from oxygen inlet points.

US5462874 discloses a multiple well tissue culture plate having a plurality of opened bottomed wells with a semi-permeable membrane across the bottoms of a plurality of such wells.

JP2001299326 describes an incubator convenient for transportation and handling.

Examples of the present disclosure seek to at least partially reduce one or more of the aforementioned problems.

### SUMMARY

According to a first aspect, a device for cellularized membrane cultivation is provided. The device comprises a culture vessel having one or more first sidewalls, a bottom surface, and a culture space being formed in an interior of the culture vessel, the culture space being configured to receive a membrane for cell and tissue growth on the bottom surface of the culture vessel. The device further comprises a cover configured to be coupled to the culture vessel, wherein the cover comprises first cover connectors and the first sidewalls of the culture vessel comprise first vessel connectors that are adapted to mate with the first cover connectors such that, in use, the first cover connectors press the membrane against the bottom surface of the culture vessel to prevent the membrane from moving.

According to this first aspect, a device that ensures keeping a membrane in place for cell and tissue growth is provided. To this end, the device comprises first cover connectors and first vessel connectors. By the simple operation of displacing the cover with respect to the vessel, the first cover connectors may be fastened to the first vessel connectors such that the first cover connectors may easily press the membrane against the bottom surface of the culture vessel. The membrane may thus be prevented from shrinking or moving and thus the cells may properly adhere to the membrane and grow.

This way, a device in which the membrane for cell cultivation may be stretched in a simple, cost-effective, and versatile way and that can be used effectively with all types of CO₂ incubators for cell culture and different kinds of membranes is provided.

In examples, in use, the attachment between the first cover connectors and the first vessel connectors define a first sealing such that culture medium and air tightness is provided.

This first sealing provides a first sealed culture space. The culture may thus be carried out of the cleanroom in substantially aseptic conditions, i.e. avoiding external contaminations and particularly during transport from a cleanroom to an operating room.

In further examples, the culture vessel further comprises one or more second sidewalls extending between a top and a bottom of the culture vessel, wherein the second sidewalls are arranged radially outwardly with respect to the first sidewalls. Radially outwardly herein is meant that the second sidewalls are further removed from the center of the vessel than the first sidewalls. The cover may further comprise second cover connectors and the second sidewalls of the culture vessel comprise second vessel connectors that are adapted to mate with the second cover connectors. In this respect, in use, the attachment between the second cover connectors and the second vessel connectors may define a second sealing such that culture medium and air tightness is provided in the culture space formed within the device.

The combination of the first sealing and second sealing (situated radially outwardly with respect to the first sealing) provide an even better sealed culture interior space. This way, the culture may be carried out in ideal aseptic conditions, i.e. avoiding external contaminations.

In a further aspect, a kit for cellularized membrane cultivation is provided. The kit comprises a device according to any of the examples herein disclosed and further comprises one or more luer - lock valves. Kits may furthermore comprise a membrane for cell growth and cultivation.

Such kits may be packaged as a unit in a sterilized packaging.

### BRIEF DESCRIPTION OF THE DRAWINGS

Non-limiting examples of the present disclosure will be described in the following, with reference to the appended drawings, in which:
Figures 1a - 1b schematically illustrate an example of a cover that can form part of a device for cellularized membrane cultivation;
Figures 2a - 2b schematically illustrate an example of a culture vessel that can form part of a device for cellularized membrane cultivation;
Figure 3 schematically illustrates an example of a device for cellularized membrane cultivation comprising a cover described in figures 1a - 1b attached to a culture vessel described in figures 2a - 2b.

### DETAILED DESCRIPTION OF EXAMPLES

Throughout the present description and claims, the term *"membrane for cell and tissue growth"* is used to refer to the growth sheet which may be provided on a cell culture vessel, where cells grow and adhere. The membrane may be biological or synthetic.

It is noted that culturing conditions, particularly the selection of culturing media, the design of culturing protocols (the supply and removal of particular media according to a particular time plan) and physical conditions such as, for example, the temperature, pressure, air humidity and lighting are not provided since they are known from conventional culturing methods and can be adapted for specific uses.

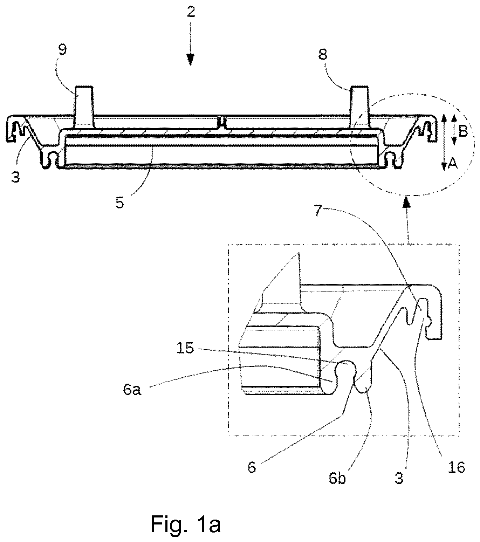

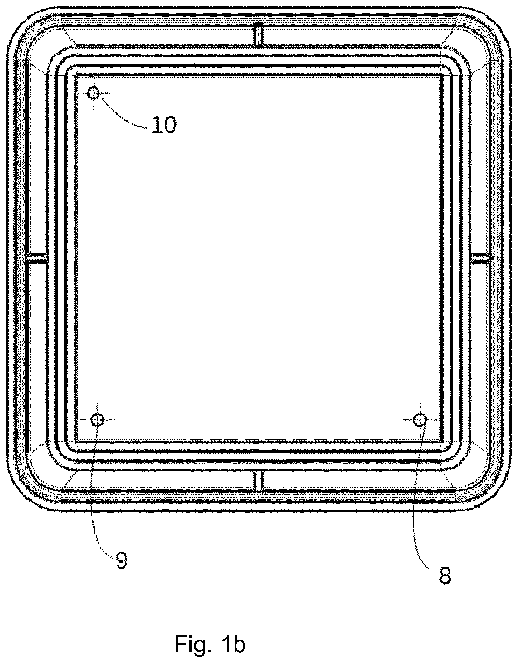

Figures 1a - 1b schematically illustrate an example of a cover which may be used in a device for cellularized membrane cultivation. Figure 1a illustrates a cross-sectional view of the cover. In this example, a cover 2 is provided. The cover 2 in this example is a substantially square cover (see figure 1b). However, other shapes of the cover are possible e.g. substantially circular-shaped. The length of each of the sides forming the square cover may be e.g. between 5 cm and 20 cm. In a particular example, the cover has a length and width of 13 cm.

The size of the culture vessel (and the corresponding cover) may be adjusted in accordance with its objective, and can thus be varied for different cell growth applications.

Following the example, the cover 2 comprises a base 5 and a "dual" twofold rim. As shown in the enlarged detail of figure 1a, the rim may comprise a first cover connector 6, a second cover connector 7 and an inclined transition portion 3 between these connectors or "coupling elements". The first cover connecter is configured to sealingly couple with a first connector on the culture vessel and the second cover connector is configured to sealingly couple with a second connector on the culture vessel as will be explained later.

In this example, both the first cover connector and the second cover connector form continuous perimeters.

The first cover connector 6 is arranged radially inwardly (i.e. closer to a geometric center of the cover) with respect to the second cover connector 7. The first cover connector 6 and the second cover connector 7 may be of constant height along their perimeters as shown in this example. As also shown in this example, the height of the first cover connector (arrow A) may be different from the height of the second cover connector (arrow B).

Particularly, the height of the first cover connector (arrow A) with respect to a top edge of the cover in this example is larger than the height of the second cover connector (arrow B).

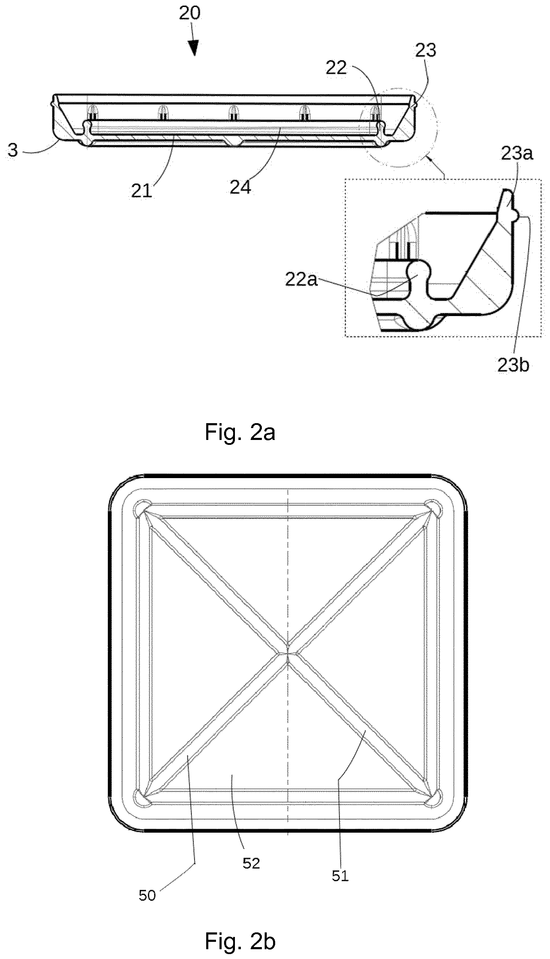

The first cover connector 6 may comprise a pair of parallel, spaced apart flanges 6a, 6b which extend substantially perpendicularly to the plane defined by the base 5. Moreover, the first cover connectors comprise a recess 15 in between the flanges 6a, 6b. Similarly, the second cover connectors 7 comprise a further recess 16. The recesses 15, 16 form female coupling members and are adapted to receive and mate with further connectors or male coupling elements located e.g. in a culture vessel, as will be explained later on. In some examples, in order to provide a sealing engagement of the male and female coupling members a snap fit may be used. In the example depicted in figures 1 and 2, the first cover connector forms a sealing clip configured to snap fit over the male connector on the culture vessel. In any case, the shape and size of the recesses 15, 16 may vary depending on the shape and size of e.g. the connectors located e.g. in a culture vessel to be attached to the cover 2. With such an arrangement, the cover 2 may be releasably joined to the culture vessel (not shown in this figure) using e.g. a snap fit locking mechanism.

Moreover, the dimensions of the cover 2 are chosen such that the cover can be coupled to the vessel 20 in a suitable way. The cover 2 may be formed of any suitable material e. g. metal, synthetic resin, glass, a polymeric material or the like, being preferably a non-porous and relatively non-fragile material capable of withstanding sterilization.

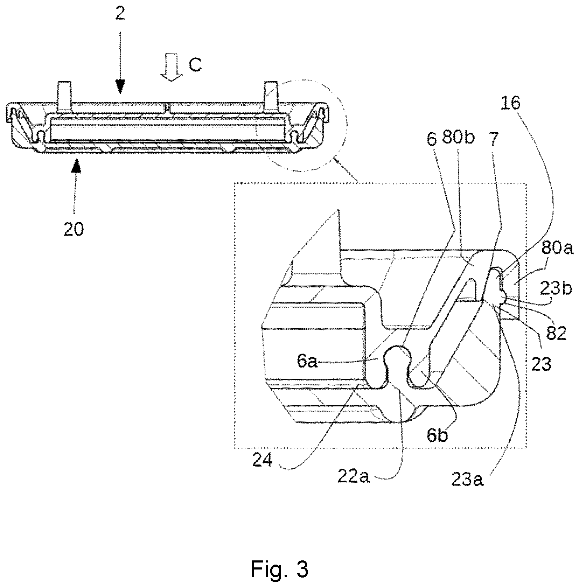

The cover 2 may comprise a grip (not shown) located e.g. at the outer part of the sidewall 3 of the cover 2, thus the attachment, removal and manipulation of the cover 2 may be improved. The grip may be shaped so that it may be easily held between the fingers of a user. The grip may also be formed by a certain surface roughness, optionally provided by a coating. With this arrangement, the cover 2 may be efficiently manipulated and used with improved accuracy. Particularly, the cover 2 may easily be axially displaced with respect to the culture vessel such that the cover is properly attached to a culture vessel and a membrane for cell cultivation is readily fixed against a bottom surface of the culture vessel, as again will be explained later on with reference to figure 3.

The cover may further be provided with one or more access ports for accessing to the space being formed in an interior of a culture vessel. Particularly, as shown in figure 1b, three access ports may be provided 8, 9 and 10. The access ports are configured in this example as truncoconical sockets. It should be clear that other recesses or sockets can be used.

The access ports 8-10 may be provided with a valve, and specifically a one-way valve. Particularly, the valves may be Luer - Lock valves. This way, e.g. a syringe may be inserted into the container body via the corresponding access port in a safe and clean manner, without contaminating the spaced formed in the interior of the vessel. Other elements such as e.g. a filter or a flexible inlet connecting tube may be connected to the corresponding access port via a Luer - lock valve. The Luer -Lock valve may be e.g. a swabable barb valve. The swabable barb valve is configured to provide a water tight hermetic seal. This valve may also be provided with a female luer lock port which provides a needleless access valve for a syringe.

For example, the access port 8 may be configured to receive and discharge a liquid e.g. growth medium or culture medium, a culture solution mixed with cells, additives, etc... The exchange of the liquid may be done using e.g. a syringe coupling with the Luer-lock valve

In some other non-illustrated examples, a flexible inlet connecting tube and / or a flexible outlet connecting tube may be provided. The connecting tubes may be connected to e.g. the access port 8 for introducing / extracting a liquid via the corresponding Luer-lock valve. The inlet tube(s) may be connected to one end to one or more irrigation bags. Fluid e.g. culture liquid may thus be introduced into the culture vessel via the inlet tube and the corresponding Luer-lock valve and fluid may be conducted out of the culture vessel via the outlet tube.

The access port 9 may be configured for a gas exchange via the corresponding Luer-lock valve. In examples, the access port 9 may be provided with a hydrophobic membrane (not shown). The hydrophobic membrane may have a pore having a diameter of, e.g. 0.5 µm or less, preferably 0.2 µm. Since a gas exchange can be performed between the inside and the outside of the culture vessel 20 using the access port 9, a gas concentration of oxygen, carbon dioxide or the like in the culture medium can be easily kept constant and / or modified. The filter may typically be made of polytetra-fluorethylene (PTFE). In some other examples, the Luer - lock valve may simply be provided with a standard filter to avoid contamination.

The access port 10 may be configured to control the pressure inside the device. In this respect, when cells or reagents are introduced inside the device, the pressure inside the device may be increased. On the contrary, when cells or reagents are extracted from the device, the pressure inside such device may be reduced. In examples, a tree-way pressure valve may be provided and connected to the access port via the corresponding Luer-lock valve. This tree-way pressure valve is configured to be closed / opened to control the pressure inside the device.

One or more three-way stopcocks (not shown) with Luer connectors may also be provided. The three-way stopcocks may be connected to the luer-lock valve of the corresponding access port. Several elements e.g. syringes may thus be connected to the same access port via the corresponding stopcock. As commented above, the means for coupling that are used for connecting the 3-way stopcock to the corresponding syringes may be universal connectors or luer-lock, which are used in medical practice. In examples, filters may also be provided in the Luer connectors of the three-way stopcocks.

Moreover, the access ports 8 - 10 may respectively be provided with a lid or stopper (not shown) so that access ports can be selectively closed / opened.

In an alternative example, instead of three access ports, only two access ports are provided. In this case, the functions of gas exchange and pressure control may be carried out through a single port, whereas the other port maybe used for the introduction of growth or culture medium.

Figures 2a - 2b schematically illustrate an example of a culture vessel which may be used in a device for cell cultivation. The culture vessel 20 shown in this example comprises a bottom inner surface 21, a first sidewall 22 and a second sidewall 23. The first sidewall 22, and the bottom surface 21 define a culturing space, which is closed off by a cover like the one shown in figure 1.

As shown in figure 2a, the second sidewall 23 is arranged radially outwardly with respect to the first sidewall 22. The sidewalls may be integrally formed with the culture vessel. It is further noted that, as depicted in the figure, the height of the second sidewall may be larger than the height of the first sidewall.

In examples, the material of the culture vessel may be different from the material of the cover (shown in figures 1a - 1b). For sealing issues, it may be beneficial that the material of the culture vessel is more rigid than the material of the cover. In any case, the employed materials may be transparent, sterilisable and biocompatible materials.

The cover (such as the one shown in figure 1) may be made of any polymeric biocompatible material suitable for sterilization e.g. liquid silicone rubber (LSR). The silicones may be used in a hardness range of 30-80 Shore A. In this particular example, the silicone used may be in a hardness range of 50 - 60 Shore A such that the cover is made softer and adaptable to a vessel. Also the water-tightness of the device may be improved. The cover may also be made using thermoplastic elastomers in a range of hardness from 50 Shore A to 60 Shore D, polyurethanes, polycarbonate (PC), polyethylene terephthalate (PET), polymethyl methacrylate (PMMA), etc.

The culture vessel may also be made of any polymeric biocompatible material suitable for sterilization e.g liquid silicone rubber (LSR). The silicones may be used in a hardness range of 30 - 80 Shore A. In this particular example, the silicone used may be in a hardness range of 70 - 80 Shore A such that the vessel is made relatively hard and rigid. The culture vessel 20 may also be made using thermoplastic elastomers in a range of hardness from 30 Shore A to 60 Shore D, polyurethanes, polycarbonate (PC), polyethylene terephthalate (PET), polymethyl methacrylate (PMMA), etc.

As shown in the enlarged detail of figure 2a, the first sidewall 22 comprises first vessel connectors 22a e.g. a male rib to sealingly couple with the female clip on the cover. Particularly, the first vessel connectors adapted to mate with the connector shown in figure 1 may be a rounded bulb portion. The rounded bulb portion 22a may define a substantially vertical plane of symmetry. The vertical plane of the rounded bulb portion may be arranged with respect to the bottom inner surface 21 of the culture vessel 20 at an angle of approximately 90 degrees. However, the first vessel connectors 22a may be tilted at different angles with respect to the plane defined by the bottom surface 21 of the culture vessel in order to improve the attachment between a cover and the culture vessel. The width of the rounded bulb portion may be e.g. 2.2 mm. In a specific example, the cover connectors may have a certain flexibility and, the width of bulb 22a may be slightly larger than recess 15 when undeformed.

Moreover, the second sidewall 23 comprises second vessel connectors. The second vessel connectors may comprise upstanding flanges 23a. At the end of the flanges 23a, bulges 23b may be provided. The flanges 23a may be elastically deformable.

In summary, the first vessel connectors 22 and the second vessel connectors 23 of the culture vessel may be adapted to mate respectively with the first cover connectors and the second cover connectors described in figures 1a - 1b. The structure and operation of the attachment between the cover and the culture vessel will be explained with more detail with reference to figure 3.

As shown in figure 2b, the culture vessel may further comprise two or more reinforcing ribs 50, 51 located at a bottom part 52 of the culture vessel.

Again in figure 2a, a cell culture membrane may also be provided at a location indicated with reference sign 24. The cell culture membrane may be disposed detachably over the bottom inner surface 21 of the culture vessel. The cell culture membrane may be a sterile biocompatible membrane previously treated with e.g. a tampon phosphate saline (PBS). It noted that, when the membrane is situated over the surface 21 of the culture vessel, air bubbles are to be avoided between the membrane and the surface 21.

The bottom surface of the culture vessel 20 may be non-cell-adhesive, and it may preferably be neither subjected to a surface treatment for an adhesive surface nor a cell-adhesive coating. In fact, the inner surface of the culture vessel 20 may be provided with a cell-repellent coating. This way, since the interior of the vessel is non-cell-adhesive, the cells to be cultivated will grow preferably in the cell culture membrane.

Figure 3 schematically illustrates an example of a device for cellularized membrane cultivation comprising a cover 2 as described in figures 1a - 1b attached to a culture vessel 20 as described in figures 2a - 2b. In this respect, the cover 2 is configured to be axially displaced in the direction of the direction of the arrow (arrow C) and coupled to the culture vessel 20, thus in use the cover closes off the top of the culture vessel 20.

The cover 2 comprises first cover connectors 6 and second cover connectors 7 as hereinbefore described with reference to figures 1a - 1b. Moreover, the culture vessel 20 comprises first vessel connectors 22a and second vessel connectors 23a as hereinbefore described, with reference to figures 2a - 2b.

As shown in the enlarged detail of figure 3, as the cover 2 is displaced in the direction of the arrow (arrow C), the rounded bulb 22a of the culture vessel may push the flanges 6a, 6b of the cover first coupling element slightly outwards. Once the rounded bulb 22a is located beyond the proximal part of the flanges, the flanges, due to the elastic deformability of such flanges, move inwards. The first cover connectors 6 and the first vessel connectors 22a are thus attached to each other.

Additionally, as the cover 2 is displaced in the direction of the arrow (arrow C), the inner flange 6a may press the cell culture membrane 24 previously located over the bottom inner surface 21 of the vessel 20. Undesired displacements of the membrane are thus avoided. It is noted that the pressing of the cell culture membrane against the bottom surface of the culture vessel is performed using an element which already forms part of the locking mechanism to attach the cover to the culture vessel. Further elements to keep the membrane in position are thus avoided.

In a specific example, in the absence of a membrane, the distal end of flange 6a may be distanced between 0 and 1 mm from the bottom surface 21. The distal end of flange 6a may even touch the bottom surface 21.

In examples, a surface defined by the membrane may be a bit larger than the bottom surface of the culture vessel over which the membrane is situated. The press of the membrane against the bottom surface of the culture vessel using the corresponding flange of the first coupling elements may thus be facilitated.

Similarly, as the cover is displaced in the direction of the arrow (arrow C), the recess 16 receives the upstanding flanges 23a and projections 80a, 80b of the second cover connector may be deformed. Once the bulges 23b of the vessel second coupling element are introduced into a recess 82, the projections 80a, 80b, due to their elastic deformability, move back to their original shape. In example, the width of the projection 80b may be e.g. 2.25 mm and its length may be e.g. 8 mm.

In summary, a snapping engagement of the first cover connectors and the second cover connectors with the first and second vessel connector may thus be achieved.

This way, a hermetic double internal and external seal is provided in the device for cultivating cells such that an aseptic closed environment wherein cells can be properly cultivated is achieved.

It is noted that a suitable culture space is formed within the device once the cover and the vessel are attached to each other. Particularly, the culture space may be provided such that, in use, a culture medium may cover the membrane and, at the same time, at least a thin layer of air may be situated between the base of the cover and the culture medium covering the membrane, to avoid the culture medium adhering to the cover. The height of the formed culture space may be e.g. between 9 and 11 mm. In this particular example, the height may be 10.7 mm.

In examples, the cover 2 and the culture vessel 20 may be sterilized and packaged in a sterile packaging, e.g. a wrap.

In accordance with an aspect, the cells can be cultivated in the device for cellularized membrane cultivation substantially as follows:
A culture vessel 20 may be provided. The culture vessel may be introduced in a cleanroom or a laminar flow cabinet which is an enclosed bench designed to prevent contamination. Additionally, a membrane impregnated with phosphate buffered saline as hereinbefore described may be provided. The membrane may be situated on the bottom surface of the vessel.

At this stage, the cover 2 may be brought into proximity of the culture vessel 20. The cover 2 may further be aligned with the culture vessel 20 and attached to the culture vessel 20, thus closing off the culture vessel 20. Firstly, the attachment between the first cover connector and the vessel first coupling element may be provided. As a result, the membrane may be kept in position over the bottom surface of the vessel as hereinbefore described. This attachment may thus provide a first seal against external pollution.

At the same time or shortly after that, the attachment between second cover connectors and the second vessel connectors may be provided. This attachment may thus provide a second seal against external pollution.

In summary, the cover and the culture vessel may be attached to each other providing a double seal against external pollution. Cells may thus be cultivated in an aseptic environment.

Following the example, a culture medium, which is a liquid for culturing cells, may be introduced into the vessel. The liquid may also comprise cells to be cultivated. The culture medium mixed with the cells may be introduced using one of the access ports with a Luer-Lock connector as hereinbefore described. This can be performed using e.g. syringe, or one or more irrigation bags connected to corresponding inlets, as indicated above.

Once the cells are introduced into the culture space in an aseptic manner, one or more of the access ports e.g. the access port configured to receive and discharge a liquid are closed off using the corresponding lid such that pollution in the interior of the culture vessel can be avoided. Moreover, the above-commented three-way pressure valve connected to the access port 10 via the corresponding Luer - lock valve may also be closed. The culture vessel 20 may be placed in e.g. an incubator (not shown) whose internal temperature e.g. 37 °c and humidity are kept constant.

At this point, the cells may grow during the necessary time until the membrane 24 is at least partially covered with grown cells. During the cells growth period, if a change in the culture medium is necessary, the previously introduced culturing medium may be extracted from the culture vessel using the corresponding port (e.g. using a syringe or an outlet line and a corresponding bag). The new culture medium may be introduced in a substantially similar way as commented above.

When the cells have been grown, the device may be transported to the desired location (e.g. an operating room). The culture medium is removed from the culture vessel and the cellularized membrane may be removed, e.g. for implantation. In examples, PBS may be introduced into the device such that the interior of the device is washed.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. A device for cellularized membrane cultivation comprising:
a culture vessel (20) having one or more first sidewalls, a bottom surface, and a culture space being formed in an interior of the culture vessel, and
a cover (2) configured to be coupled to the culture vessel, wherein
the culture space is configured to receive a membrane for cell and tissue growth on the bottom surface of the culture vessel, **characterized in that**
the cover (2) comprises first cover connectors (6) and the first sidewalls (22) of the culture vessel (20) comprise first vessel connectors (22a) that are adapted to mate with the first cover connectors (6) such that, in use, the first cover connectors (6) press the membrane against the bottom surface of the culture vessel to prevent the membrane (24) from moving.

2. A device according to claim 1, wherein the first vessel connectors (22a) comprise a male rib and the first cover connectors (6) comprise a female sealing clip configured to snap fit over the rib.

3. A device according to claim 2, wherein the female sealing clip comprises two flanges configured to snap fit around a bulb portion on the rib, wherein one of the flanges presses the membrane against the bottom surface of the culture vessel (20).

4. A device according to any of claims 1 - 3, wherein the first cover connectors (6) are arranged at an angle of 90 degrees with respect to a plane defined by the bottom surface of the culture vessel (20).

5. A device according to any of claims 1 - 4, wherein the culture vessel (20) further comprises one or more second sidewalls (23) extending between a top and a bottom of the culture vessel (20), wherein the second sidewalls (23) are arranged radially outwardly with respect to the first sidewalls (22).

6. A device according to claim 5, wherein a height of the first sidewalls is smaller than a height of the second sidewalls (23).

7. A device according to any of claims 5 - 6, wherein the first sidewalls (22) and the second sidewalls (23) are integrally formed with the culture vessel (20).

8. A device according to any of claims 1 - 7, wherein, in use, the attachment between the first cover connectors (6) and the first vessel connectors (22a) define a first sealing such that water and air tightness is provided by the first sealing.

9. A device according to any of claims 5 - 8, wherein the cover (2) further comprises second cover connectors (7) and the second sidewalls (23) of the culture vessel comprise second vessel connectors (23a) that are adapted to mate with the second cover connectors (7).

10. A device according to claim 9, wherein the second vessel connectors (23a) comprise one or more upstanding flanges, the flanges having bulges at or near their ends and wherein the second cover connectors (7) are configured to mate with the flanges by snapping over the bulges.

11. A device according to any of claims 9 -10, wherein, in use, the attachment between the second cover connectors (7) and the second vessel connectors (23a) define a second sealing such that water and air tightness is provided by the second sealing.

12. A device according to any of claims 1 - 11, wherein the cover (2) is a substantially square cover and the culture vessel is a substantially square culture vessel, wherein the coupling elements are configured for an axial movement of the cover with respect to the vessel during coupling.

13. A device according to any of claims 1 - 12, wherein the cover further comprises one or more access ports for accessing the culture space.

14. A device according to claim 13, wherein one or more of the access ports (8 -10)are suitable for gas exchange in the culture space and / or one or more of the access ports are suitable for the extraction and introduction of a liquid in the culture space and / or one or more of the access ports are suitable to control the pressure inside the device

15. A kit for cellularized membrane cultivation comprising a device for cellularized membrane cultivation according to any of claims 1 - 14, and further comprising one or more luer - lock valves.

## Patentansprüche

1. Eine Vorrichtung zur Kultivierung von zellularisierten Membranen, umfassend:
ein Kulturgefäß (20) mit einer oder mehreren ersten Seitenwänden, einer Bodenfläche und einem Kulturraum, der in einem Inneren des Kulturgefäßes gebildet ist, und
eine Abdeckung (2), die konfiguriert ist, um an das Kulturgefäß gekoppelt zu werden, wobei
der Kulturraum konfiguriert ist, um eine Membran für das Zell- und Gewebewachstum auf der Bodenfläche des Kulturgefäßes aufzunehmen, **dadurch gekennzeichnet, dass**
die Abdeckung (2) erste Abdeckungsverbindungselemente (6) umfasst und die ersten Seitenwände (22) des Kulturgefäßes (20) erste Gefäßverbindungselemente (22a) umfassen, die so angepasst sind, dass sie mit den ersten Abdeckungsverbindungselementen (6) zusammenpassen, so dass die ersten Abdeckungsverbindungselemente (6) im Einsatz die Membran gegen die Bodenfläche des Kulturgefäßes drücken, um zu verhindern, dass sich die Membran (24) bewegt.

2. Eine Vorrichtung nach Anspruch 1, wobei die ersten Gefäßverbindungselemente (22a) eine männliche Rippe umfassen und die ersten Abdeckungsverbindungselemente (6) einen weiblichen Dichtungsclip umfassen, der konfiguriert ist, um über die Rippe zu schnappen.

3. Eine Vorrichtung nach Anspruch 2, wobei der weibliche Dichtungsclip zwei Flansche umfasst, die konfiguriert sind, um einen Wulstabschnitt an der Rippe einzuschnappen, wobei einer der Flansche die Membran gegen die Bodenfläche des Kulturgefäßes (20) drückt.

4. Eine Vorrichtung nach einem der Ansprüche 1 bis 3, wobei die ersten Abdeckungsverbindungselemente (6) in einem Winkel von 90 Grad in Bezug auf eine durch die Bodenfläche des Kulturgefäßes (20) definierte Ebene angeordnet sind.

5. Eine Vorrichtung nach einem der Ansprüche 1 bis 4, wobei das Kulturgefäß (20) weiterhin eine oder mehrere zweite Seitenwände (23) umfasst, die sich zwischen einem Oberteil und einem Boden des Kulturgefäßes (20) erstrecken, wobei die zweiten Seitenwände (23) in Bezug auf die ersten Seitenwände (22) radial nach außen angeordnet sind.

6. Eine Vorrichtung nach Anspruch 5, wobei eine Höhe der ersten Seitenwände kleiner ist als eine Höhe der zweiten Seitenwände (23).

7. Eine Vorrichtung nach einem der Ansprüche 5 bis 6, wobei die ersten Seitenwände (22) und die zweiten Seitenwände (23) einstückig mit dem Kulturgefäß (20) ausgebildet sind.

8. Eine Vorrichtung nach einem der Ansprüche 1 bis 7, wobei die Befestigung zwischen den ersten Abdeckungsverbindungselementen (6) und den ersten Gefäßverbindungselementen (22a) im Einsatz eine erste Abdichtung definiert, so dass durch die erste Abdichtung Wasser- und Luftdichtheit bereitgestellt wird.

9. Eine Vorrichtung nach einem der Ansprüche 5 bis 8, wobei die Abdeckung (2) weiterhin zweite Abdeckungsverbindungselemente (7) umfasst und die zweiten Seitenwände (23) des Kulturgefäßes zweite Gefäßverbindungselemente (23a) umfassen, die angepasst sind, um mit den zweiten Abdeckungsverbindungselementen (7) zusammenzupassen.

10. Eine Vorrichtung nach Anspruch 9, wobei die zweiten Gefäßverbindungselemente (23a) einen oder mehrere hochstehende Flansche umfassen, wobei die Flansche Ausbuchtungen an oder in der Nähe von ihren Enden haben und wobei die zweiten Abdeckungsverbindungselemente (7) konfiguriert sind, um mit den Flanschen durch Schnappen über die Ausbuchtungen zusammenzupassen.

11. Eine Vorrichtung nach einem der Ansprüche 9 bis 10, wobei die Befestigung zwischen den zweiten Abdeckungsverbindungselementen (7) und den zweiten Gefäßverbindungselementen (23a) im Einsatz eine zweite Abdichtung definiert, so dass durch die zweite Abdichtung Wasser- und Luftdichtheit bereitgestellt wird.

12. Eine Vorrichtung nach einem der Ansprüche 1 bis 11, wobei die Abdeckung (2) eine im Wesentlichen quadratische Abdeckung ist und das Kulturgefäß ein im Wesentlichen quadratisches Kulturgefäß ist, wobei die Kupplungselemente für eine axiale Bewegung der Abdeckung in Bezug auf das Gefäß während der Kopplung konfiguriert sind.

13. Eine Vorrichtung nach einem der Ansprüche 1 bis 12, wobei die Abdeckung weiterhin eine oder mehrere Zugangsöffnungen für den Zugang zum Kulturraum umfasst.

14. Eine Vorrichtung nach Anspruch 13, wobei eine oder mehrere der Zugangsöffnungen (8 - 10) zum Gasaustausch im Kulturraum geeignet sind und / oder eine oder mehrere der Zugangsöffnungen zur Entnahme und Einführung von einer Flüssigkeit in den Kulturraum geeignet sind und / oder eine oder mehrere der Zugangsöffnungen zur Regelung des Drucks im Inneren der Vorrichtung geeignet sind.

15. Ein Kit zur Kultivierung von zellularisierten Membranen, umfassend eine Vorrichtung zur Kultivierung von zellularisierten Membranen nach einem der Ansprüche 1 bis 14, und weiterhin umfassend ein oder mehrere Luer-Lock-Ventile.

## Revendications

1. Un dispositif de culture de membranes cellularisées comprenant :
une cuve de culture (20) ayant une ou plusieurs premières parois latérales, une surface inférieure et un espace de culture étant formé dans un intérieur de la cuve de culture, et
un couvercle (2) configuré pour être couplé à la cuve de culture, dans lequel
l'espace de culture est configuré pour recevoir une membrane pour la croissance cellulaire et tissulaire sur la surface inférieure de la cuve de culture, **caractérisé en ce que**
le couvercle (2) comprend des premiers connecteurs de couvercle (6) et les premières parois latérales (22) de la cuve de culture (20) comprennent des premiers connecteurs de cuve (22a) qui sont adaptés pour s'accoupler avec les premiers connecteurs de couvercle (6) de telle sorte que, lors du fonctionnement, les premiers connecteurs de couvercle (6) pressent la membrane contre la surface inférieure de la cuve de culture pour empêcher le mouvement de la membrane (24).

2. Un dispositif selon la revendication 1, dans lequel les premiers connecteurs de cuve (22a) comprennent une nervure mâle et les premiers connecteurs de couvercle (6) comprennent une agrafe de fermeture femelle configurée pour être emboîtée sur la nervure.

3. Un dispositif selon la revendication 2, dans lequel l'agrafe de fermeture femelle comprend deux brides configurées pour être emboîtées autour d'une portion de bulbe sur la nervure, dans lequel l'une des brides presse la membrane contre la surface inférieure de la cuve de culture (20).

4. Un dispositif selon l'une quelconque des revendications 1 à 3, dans lequel les premiers connecteurs de couvercle (6) sont disposés à un angle de 90 degrés par rapport à un plan défini par la surface inférieure de la cuve de culture (20).

5. Un dispositif selon l'une quelconque des revendications 1 à 4, dans lequel la cuve de culture (20) comprend en outre une ou plusieurs secondes parois latérales (23) s'étendant entre un haut et un fond de la cuve de culture (20), dans lequel les secondes parois latérales (23) sont disposées radialement vers l'extérieur par rapport aux premières parois latérales (22).

6. Un dispositif selon la revendication 5, dans lequel une hauteur des premières parois latérales est inférieure à une hauteur des secondes parois latérales (23).

7. Un dispositif selon l'une quelconque des revendications 5 à 6, dans lequel les premières parois latérales (22) et les secondes parois latérales (23) sont formées d'un seul tenant avec la cuve de culture (20).

8. Un dispositif selon l'une quelconque des revendications 1 à 7, dans lequel, lors du fonctionnement, la fixation entre les premiers connecteurs de couvercle (6) et les premiers connecteurs de cuve (22a) définit un premier scellage tel que de l'étanchéité à l'eau et à l'air est fournie par le premier scellage.

9. Un dispositif selon l'une quelconque des revendications 5 à 8, dans lequel le couvercle (2) comprend en outre des seconds connecteurs (7) de couvercle et les secondes parois latérales (23) de la cuve de culture comprennent des seconds connecteurs de cuve (23a) qui sont adaptés pour s'accoupler avec les seconds connecteurs de couvercle (7).

10. Un dispositif selon la revendication 9, dans lequel les seconds connecteurs de cuve (23a) comprennent une ou plusieurs brides verticales, les brides ayant des renflements à ou près de leurs extrémités et dans lequel les seconds connecteurs de couvercle (7) sont configurés pour être accouplés avec les brides en les encliquetant sur les renflements.

11. Un dispositif selon l'une quelconque des revendications 9 à 10, dans lequel, lors du fonctionnement, la fixation entre les seconds connecteurs de couvercle (7) et les seconds connecteurs de cuve (23a) définit un second scellage tel que de l'étanchéité à l'eau et à l'air est fournie par le second scellage.

12. Un dispositif selon l'une quelconque des revendications 1 à 11, dans lequel le couvercle (2) est un couvercle essentiellement carré et la cuve de culture est une cuve de culture essentiellement carrée, dans lequel les éléments de couplage sont configurés pour un mouvement axial du couvercle par rapport à la cuve pendant le couplage.

13. Un dispositif selon l'une quelconque des revendications 1 à 12, dans lequel le couvercle comprend en outre un ou plusieurs orifices d'accès pour accéder à l'espace de culture.

14. Un dispositif selon la revendication 13, dans lequel un ou plusieurs des orifices d'accès (8 - 10) sont appropriés pour l'échange gazeux dans l'espace de culture et / ou un ou plusieurs des orifices d'accès sont appropriés pour l'extraction et l'introduction d'un liquide dans l'espace de culture et / ou un ou plusieurs des orifices d'accès sont appropriés pour contrôler la pression à l'intérieur du dispositif.

15. Un kit pour la culture de membranes cellularisées comprenant un dispositif pour la culture de membranes cellularisées selon l'une quelconque des revendications 1 à 14, et comprenant en outre une ou plusieurs soupapes de type luer - lock.
